Europäisches Patentamt

European Patent Office

Office européen des brevets

(11) Publication number: **0 205 303 B1**

(12) **EUROPEAN PATENT SPECIFICATION**

(45) Date of publication of patent specification: **29.07.92**  (51) Int. Cl.⁵: **C12P 5/02**

(21) Application number: **86304221.4**

(22) Date of filing: **03.06.86**

(54) **Microbiological method of producing ethylene.**

(30) Priority: **06.06.85 JP 123118/85**

(43) Date of publication of application:
**17.12.86 Bulletin 86/51**

(45) Publication of the grant of the patent:
**29.07.92 Bulletin 92/31**

(84) Designated Contracting States:
**BE CH DE FR GB IT LI NL SE**

(56) References cited:
**EP-A- 0 178 153**

**AGRIC. BIOL. CHEM., vol. 50, no. 4, April 1986, pages 977-981; H. FAKUDA et al.: "preparation of a cell-free ethylene-forming system form penicillium digitatum"**

**CHEMICAL ABSTRACTS, vol. 102, 1985, page 490, abstract no. 165183z, Columbus, Ohio, US; H. FAKUDA: "Ethylene production with microorganisms" & KAGAKU TO SEIBUTSU 1984, 22(11), 752-3**

**ARCHIVES OF BIOCHEMISTRY AND BIOPHYSICS, vol. 157, 1973, pages 73-82, Academic Press Inc.; T.W. CHOU et al.: "The biogenesis of ethylene in penicillium digitatum"**

(73) Proprietor: **Fukuda, Hideo**
**5-10, Tezukayama-naka 3-chome**
**Sumiyoshi-ku Osaka 558(JP)**

(72) Inventor: **Fukuda, Hideo**
**5-10, Tezukayama-naka 3-chome Sumiyoshi-ku**
**Osaka 558(JP)**
Inventor: **Ogawa, Takahira**
**20-2, Ikeda 3-chome**
**Kumamoto 860(JP)**
Inventor: **Fujii, Takao**
**10-5, Ikeda 3-chome**
**Kumamoto 860(JP)**

(74) Representative: **Raynor, John et al**
**W.H. Beck, Greener & Co 7 Stone Buildings Lincoln's Inn**
**London WC2A 3SZ(GB)**

## Description

The present invention relates to a method of producing ethylene which comprises cultivating a fungal organism.

Regarding the production of ethylene by micro-organisms, an investigational research on 228 species of molds [L. Ilag et al[2], Science 159, 1357-1358 (1968)], researches on Mucor sp. and Aspergillus clavatus [J. M. Lynch[3], Nature 240, 45-46 (1972); J. M. Lynch and S. H. Harper[4], J. Gen. Microbiol. 80, 187-195 (1974)-], an investigational research on soil bacteria [S. B. Primrose[5], J. Gen. Microbiol. 97, 343-346 (1976)], researches on Escherichia coli and Pseudomonas sp. [S. B. Primrose[6], J. Gen. Microbiol. 95, 159-165 (1976); S. B. Primrose et al[7], J. Gen. Microbiol. 93, 177-181 (1976); H. T. Freebairn et al[8], Nature 202, 313-314 (1964)], a research on Saccharomyces cerevisiae [K. C. Thomas et al[9], Can. J. Microbiol. 23, 1669-1674 (1977)], a research on mushrooms [E. M. Turner[10], J. Gen. Microbiol. 91, 167-176 (1975)], and a review of these researches [M. Lieberman[11], Ann. Rev. Plant Physiol. 30, 533-591 (1979)], for instance, have been conducted. However, most of these reports are only investigational in nature, and there has been no significant discussion on the kinds of microorganisms capable of producing ethylene.

Regarding the production of ethylene by organisms of the genus Penicillium, the above-mentioned report of Ilag et al[2] refers to P. corylophilum, P. luteum end P. patulum. The production of ethylene by Penicillium digitatum is mentioned in the above-mentioned reports of Lieberman[11] and D. L. Ketring et al[12] [Plant Cell Physiol. 9, 617 (1968)] without indication of particular strain numbers, and Spalding et al[13] [Plant Physiol. 40, 645 (1965)] mentions ATCC 10030 strain. However, all of these disclosures are merely investigational and do not provide information on an established procedure for ethylene production.

Regarding the pathways of ethylene biosynthesis, the pathway involving methionine in plants has been elucidated by D. O. Adams et al[14] [Proc. Nat. Acad. Sci., U.S.A. 76, 170 (1979)] but the pathway of ethylene biosynthesis by microorganisms remains yet to be established. For example, as precursors of ethylene biosynthesis by P. digitatum, D. W. Jacobson et al[15] [Plant Physiol. 43, 1959 (1968)] referred to acrylic acid, D. L. Ketring et al to isocitric acid, T. W. Chou et al[16] [Arch. Biochem. Biophys. 157, 73 (1973)] to 2-ketoglutaric acid and L-glutamic acid, and E. Chalutz et al[17] [Plant Physiol. 60, 402 (1977)] to L-methionine. Incidentally, Chou et al[16] failed to identify which of 2-ketoglutaric acid and L-glutamic acid is the true precursor.

As regards Mucor sp. , there is the above-mentioned report[4] suggesting that methionine is involved in the production of ethylene by Mucor hiemalis.

The fact that the biosynthetic precursors of ethylene mentioned by these reporters are not uniform is clear evidence that the pathway of ethylene biosynthesis in microorganisms has not been established.

As mentioned above, studies on the production of ethylene by microorganisms are still in the exploratory stage and there is no established theory about its biosynthetic pathway. For that matter, there is not known a method that can be practiced on an industrial scale to manufacture ethylene with the aid of micro-organisms with sufficient reproducibility.

The present inventors carried out a detailed investigation about the process of ethylene biosynthesis in microorganisms and obtained the following experimental results.

Experiment 1 Response of ethylene-producing strains to L-methionine:

Using ethylene-producing microorganisms, namely 8 bacterial strains, 10 yeast strains and 7 fungal strains, their production rates of ethylene were measured by gas chromatography. Some of the data are shown in Table 2. In this experiment, the responses of the strains to L-methionine were compared by growing them in the L-methionine-containing media shown in Table 1 and the corresponding L-methionine-free media. It was found that irrespective of a taxonomic distinction of bacteria, yeasts and fungi, all of the 24 ethylene-producing strains tested responded to L-methionine with the exception of Penicillium digitatum IFO 9372 which failed to respond to this amino acid.

### Table 1 Media and cultural conditions
### for ethylene-producing strains

(Unit: g/l)

| Media and cultural conditions | Bacteria | Yeasts | Fungi | Modified NB medium |
|---|---|---|---|---|
| Glucose | 20 | 20 | 40 | 40 |
| $(NH_4)_2SO_4$ | 5 | 5 | 3 | − |
| $K_2HPO_4$ | 1 | 1 | 1 | − |
| $MgSO_4 \cdot 7H_2O$ | 0.5 | 0.5 | 0.5 | − |
| $CaCl_2 \cdot 2H_2O$ | 0.1 | 0.1 | 0.1 | − |
| NaCl | 0.1 | 0.1 | 0.1 | 2 |
| $FeSO_4 \cdot 7H_2O$ | − | − | 0.01 | 0.01 |
| KCl | − | − | 0.25 | − |
| $ZnSO_4 \cdot 7H_2O$ | − | − | 0.22 | − |
| $CaCO_3$ | − | 3 | 3 | − |
| Meat extract | − | − | − | 3 |
| Yeast extract | 5 | 5 | − | 2 |
| Polypeptone | − | − | − | 5 |
| Inorganic salt soln.* | 10 ml/ℓ | 10 ml/ℓ | − | − |
| C.M.C.** | − | − | 30 | − |
| pH | 7.0 | 5.5 | 6.0 | 6.0 |
| Incubation temperature | 30°C | 25°C | 25°C | |
| Cultural method | Rotary shaker method | | Rotary shaker method | |
| Duration (in days) of cultivation | 2 days | 2 days | 4-5 days | |

\* Inorganic salt soln.: Sodium citrate 5 g/$\ell$, $MnCl_2 \cdot 4H_2O$ 3 g/$\ell$, $ZnCl_2$ 2 g/$\ell$, $FeCl_3 \cdot 6H_2O$ 2 g/$\ell$, $CuSO_4 \cdot 5H_2O$ 0.2 g/$\ell$ $CoCl_2 \cdot 6H_2O$ 0.2 g/$\ell$, $Na_2MoO_4 \cdot 2H_2O$ 0.1 g/$\ell$, $K_2B_4O_7 \cdot xH_2O$ 0.1 g/$\ell$

\*\* C.M.C.: Carboxymethylcellulose (sodium salt)

Table 2  Responses of ethylene-producing strains to methionine

Unit: nl/ml (culture broth)/hr.

| Strain | | Without L-methionine | With L-methionine |
|---|---|---|---|
| Corynebacterium aquaticum | IFO12154 | 0.15 | 0.65 |
| " paurometabolum | IFO12160 | – | 0.52 |
| Bacillus licheniformis | IFO12107 | 0.15 | 0.30 |
| Pseudomonas putida | IFO 3738 | 0.16 | 0.80 |
| Schizosaccharomyces octosporus | IFO 0353 | 0.20 | 5.20 |
| Cryptococcus laurentii | IFO 0384 | 0.05 | 1.20 |
| " albidus | IFO 1320 | 0.05 | 13.60 |
| Debaryomyces hansenii | IFO 0015 | 0.10 | 2.50 |
| Aspergillus clavatus | IFO 8606 | 0.05 | 0.20 |
| Monilia geophila | IFO 5425 | 0.05 | 0.30 |
| Nectria flammea | IFO 9628 | 0.10 | 5.10 |
| Paecilomyces elegans | IFO 6619 | 0.05 | 1.00 |
| Rhizopus japonicus | IFO 4758 | 0.10 | 2.60 |
| Penicillium digitatum | IFO 9372 | 21.4 | 19.6 |

The level of addition of L-methionine· 1 g/$\ell$

Experiment 2 ACC* synthase activity and ethylene production rate

The activity of ACC synthase, a representative enzyme in the methionine pathway in plants, was determined in P. digitatum IFO 9372 which failed to respond to L-methionine (Table 2 above) and Cry. albidus IFO 1320 which showed the highest ethylene production rate of all the 24 strains (Table 2). The results are shown in Table 3. In P. digitatum IFO 9372, a large quantity of ethylene was produced notwithstanding the fact that ACC synthase activity was not detected. To measure the ACC synthase activity, a cell-free extract from each culture broth was reacted with S-adenosylmethionine as the substrate and the ethylene evolved on addition of sodium hypochlorite was determined by gas chromatography.

Table 3  ACC* synthase activity and ethylene production rate

| Strain | ACC synthase activity* (n mol/mg protein/hr) | Ethylene production rate (nl/ml culture broth/hr) |
|---|---|---|
| Penicillium digitatum IFO 9372 | ND** | 297 |
| Cryptococcus albidus IFO 1320 | $2.4 \times 10^{-3}$ | 42 |

\*   ACC:  1-Aminocyclopropane-1-carboxylic acid

\*\*  ND :  Not detected

Both strains were cultivated in the modified NB medium of Table 1 in the routine manner.

Experiment 3 Influence of addition of various substances to the culture broth of P. digitatum IFO 9372

As it was expected that unlike most other ethylene-producers, P. digitatum IFO 9372 produces ethylene by a route other than the methionine pathway, the effects of addition of various compounds that might be precursors were investigated. The cells of P. digitatum grown in the modified NB medium of Table 1 at

6

25°C for 3 days in the routine manner were washed and resuspended in 0.1 M phosphate buffer (pH 6.0). On the 4th day of starvation culture, various compounds were added and the rates of ethylene production by resting cells were determined. The results are given in Table 4. The effect of L-glutamic acid was greatest, while the addition of L-methionine and other compounds had substantially no effect.

Table 4

| Influence of addition of various compounds to washed resting cells of P. digitatum IFO 9372 | |
| --- | --- |
| Additive | Ethylene production rate (nl/ml reaction medium/hr) |
| Sodium L-glutamate | 8.8 |
| L-Alanine | 5.3 |
| Succinic acid semialdehyde | 5.2 |
| $\gamma$-Aminobutyric acid | 4.9 |
| Acrylic acid | 4.2 |
| L-Methionine | 1.3 |
| No addition | 2.8 |

Resting cells:

Washed cells after 4 days of starvation culture were used.

Reaction conditions:

Concentration of resting cells:  18 mg/ml reaction medium
Concentration of additives:  1 mM (per reaction medium)
Reaction conditions:  25°C, reciprocating shaker method, 24 hr.

Experiment 4 Extraction and purification of the ethylene-producing enzyme of P. digitatum IFO9372

The cells of P. digitatum grown in the modified NB medium of Table 1 in the routine manner were washed and treated in the sequence shown in Table 5 to extract, purify and isolate the ethylene-producing enzyme.

Table 5   Flow chart for extraction and purification of

ethylene-producing enzyme

Culture broth of P. digitatum IFO9372

  |  ←—— Filter and wash
  ↓
Washed cells

  |  ←—— Homogenize with marine
  |       sand and suspend in buffer
  ↓
Homogenate

  |  ←—— Centrifuge
  ↓
Cell-free extract [A]

  |  ←—— Salt out with ammonium sulfate (40-60%)
  |  ←—— Dialyze
  ↓
Crude enzyme solution [B]

  |  ←—— Ion exchange with DEAE-Sepharose CL-6B
  ↓

Effluent                   |—— Elute with 0.1-0.15 M NaCl
fraction [C]               ↓

       Ethylene-producing enzyme fraction [D]

Experiment 5 Influence of addition of various compounds to cell-free extract [A]

Several compounds that were considered to serve as substrates were added to the cell-free extract [A] of Table 5 and the ethylene production rates were determined. As shown in Table 6, the ethylene production rate was highest with $\alpha$-ketoglutaric acid while there was substantially no production of ethylene in the presence of L-glutamic acid or L-methionine. The intrinsic substrate for ethylene-producing enzyme seemed to be $\alpha$-ketoglutaric acid.

Table 6

| Influence of addition of various compounds to cell-free extract [A] | |
| --- | --- |
| Additive | Ethylene production rate (nl/ml reaction medium/hr) |
| $\alpha$-Ketoglutaric acid | 27.31 |
| Succinic acid semi aldehyde | 2.90 |
| S-Adenosylmethionine | 0.79 |
| L-Glutamic acid | 0.76 |
| Sodium L-glutamate | 0.54 |
| 1-Aminocyclopropane-1-carboxylic acid | 0.52 |
| Succinic acid | 0.47 |
| $\beta$-Alanine | 0.43 |
| $\gamma$-Amino-n-butyric acid | 0.42 |
| L-Methionine | 0.36 |
| L-Alanine | 0.30 |
| No addition | 0.52 |

Reaction conditions:

Cell-free extract [A]:

the final concentration of protein in the reaction system: 3.32 mg/ml (reaction medium)

Concentration of additives:

1 mM (per reaction medium)

Reaction conditions:

25°C, reciprocating shaker method, 1 hr.

Experiment 6 influence of addition of metal salts to crude enzyme solution [B]

Using the crude enzyme solution [B] of Table 5, obtained by ammonium sulfate fractionation and subsequent dialysis of cell-free extract [A], an enzymatic reaction with the substrate $\alpha$-ketoglutaric acid was conducted. As shown in Table 7, when there was no additive agent, the ethylene production rate decreased to about 1/10 as compared with the case of Table 6 where $\alpha$-ketoglutaric acid was added. Therefore, in order to clarify the factor lost by dialysis, an experiment was performed using the metal salts given in Table 7. As a result, it was found that divalent iron ion is necessary for activity of the ethylene-producing enzyme. The optimum level of addition of ferrous sulfate was within the range of 0.05 mM to 0.3 mM.

Table 7

| Influence of addition of metal salts to crude enzyme solution [B] | |
|---|---|
| Metal salt added | Ethylene production rate (nl/mg protein/hr) |
| No addition | 0.26 |
| $FeSO_4 \cdot 7H_2O$ | 5.21 |
| $Al_2(SO_4)_3$ | 0.51 |
| $Fe_2(SO_4)_3 \cdot xH_2O$ | 0.50 |
| $K_2SO_4$ | 0.29 |
| $K_2B_4O_7 \cdot xH_2O$ | 0.26 |
| $(NH_4)_6Mo_7O_{24} \cdot 4H_2O$ | 0.24 |
| $CaCl_2$ | 0.23 |
| NaCl | 0.23 |
| $MgSO_4 \cdot 7H_2O$ | 0.17 |
| $CuSO_4 \cdot 5H_2O$ | 0.15 |
| $MnSO_4 \cdot 4\text{-}5H_2O$ | 0.13 |
| $ZnSO_4 \cdot 7H_2O$ | 0 |
| $CoSO_4 \cdot 7H_2O$ | 0 |
| $NiSO_4 \cdot 6H_2O$ | 0 |

Reaction conditions:

Crude enzyme solution [B]:

The final concentration of protein in reaction meduim: 3.47 mg/ml reaction medium
The final concentration of substrate $\alpha$-ketoglutaric acid: 1 mM
The final concentration of metal salt: 0.1 mM
Reaction conditions: 25°C, reciprocating shaker method, 1hr.

Experiment 7 Ethylene production with DEAE-Sepharose fractions [C] and [D]

To the effluent fraction [C] from the DEAE-Sepharose CL-6B ion exchange column [See Table 5] was added $\alpha$-ketoglutaric acid as the substrate, and the enzymatic reaction was conducted in the presence of ferrous sulfate as the divalent iron ion necessary for the reaction. As apparent from Table 8, there was no production of ethylene.

The substances adsorbed on the DEAE-Sepharose CL-6B ion exchange column were eluted on a sodium chloride gradient, and the ethylene-producing enzyme fraction [D] emerging at about 0.15 mol NaCl was reacted with substrate $\alpha$-ketoglutaric acid in the presence of ferrous sulfate necessary for the reaction. As shown in Table 8, only a small amount of ethylene was produced.

The above fractions [C] and [D] were admixed in equal proportions and the mixture was reacted with $\alpha$-ketoglutaric acid in the presence of ferrous sulfate necessary for the reaction. As apparent from Table 8, the production of ethylene was observed.

As the chief component of fraction [C] is a low molecular weight substance which gave a positive ninhydrin reaction, it was suspected to be an amino acid. Accordingly, fraction [D] was admixed with casamino acid instead of fraction [C] and the enzymatic reaction was conducted. As apparent from Table 8, a large quantity of ethylene was produced. It appears, therefore, that fraction [C] is an amino acid and that a substantial proportion of this active component was lost by ammonium sulfate fractionation and dialysis.

Table 8  Ethylene production with DEAE-Sepharose fractions [C] and [D]

| Fraction | Ethylene production rate (nl/ml reaction medium/hr) |
|---|---|
| [C]* | 0 |
| [D]** | 7.3 |
| [C] + [D]*** | 162.4 |
| [Casamino acid] + [D]**** | 1327.1 |

Reaction conditions:

    * Fraction [C]:  The final concentration of protein

                    in reaction system: 0.59 mg/ml

                                        reaction medium

    ** Fraction [D]:  The final concentration of protein

                    in reaction system: 0.95 mg/ml

                                        reaction medium

*** Mixing ratio of fraction[C] to
    Fraction [D]: 1 vol./1 vol.(**)

**** Casamino acid: The final concentration of casamino
     acid in reaction system: 2 mg/ml reaction medium

    The final concentration of $\alpha$-ketoglutaric acid: 1 mM

    The final concentration of ferrous sulfate: 0.075 mM

    Reaction conditions: 25°C, reciprocating shaker method,

                        1 hr

    (**): Composition of reaction system:

    Fraction [C] + Fraction [D] +
        (0.2 ml)        (0.2 ml)

    HEPES buffer +   $\alpha$-KG   +   $FeSO_4.7H_2O$
        (pH 8.0)      (10 mM)      (0.575 mM)
        (0.4 ml)     (0.1 ml)      (0.1 ml)

Experiment 8 The effects of addition of amino acids to ethylene-producing enzyme fraction [D]

Instead of adding Fraction [C] or casamino acid, a variety of amino acids were respectively added to ethylene-producing enzyme fraction [D] of Table 5 and the enzymatic reaction was conducted using substrate α-ketoglutaric acid in the presence of ferrous sulfate necessary for the reaction. As apparent from Table 9, a remarkable quantity of ethylene was produced when L-arginine was added.

Therefore, L-arginine in the aforementioned effluent fraction [C] was determined. As a result, about 0.05 mM of L-arginine was detected.

Based on the above experimental results, the active component of Fraction [C] was established to be L-arginine. While such low molecular substances should have been lost in the course of dialysis in the purification process of Table 5, it appears that L-arginine was adsorbed on the enzyme protein and remained in the fraction.

Table 9

| Effects of addition of amino acids to ethylene-producing enzyme fraction [D] | |
|---|---|
| Amino acid added | Ethylene production rate (nl/mg protein/ hr.) |
| No addition | 7.3 |
| L-Arginine | 422.2 |
| L-Histidine | 18.1 |
| L-Threonine | 11.5 |
| L-Valine | 11.4 |
| L-Cysteine | 9.7 |
| L-Glutamine | 9.0 |
| L-Proline | 8.9 |
| L-Aspartic acid | 8.9 |
| L-Leucine | 8.8 |
| L-Methionine | 8.4 |
| Sodium L-glutamate | 8.3 |
| Glycine | 8.2 |
| L-Isoleucine | 7.9 |
| L-Lysine | 7.6 |
| L-Asparagine | 7.1 |
| L-Tryptophan | 7.1 |
| L-Serine | 7.0 |
| L-Tyrosine | 7.0 |
| L-Phenylalanine | 6.8 |
| L-Alanine | 6.6 |
| L-Hydroxyproline | 6.6 |

Reaction conditions:

The level of addition of Fraction [D]:

The final protein concentration in reaction system: 0.1 mg/ml reaction medium
The final concentration of substrate α-ketoglutaric acid: 1 mM,
The final concentration of ferrous sulfate: 0.075 mM.
The final concentration of each amino acid: 2 mM
The reaction was conducted at 25°C for 1 hr.

Based on the above experimental results, an in vitro system for ethylene production using α-ketoglutaric acid as the substrate was established as follows. Thus, a reaction mixture of 0.1 ml of 10 mM α-ketoglutaric acid, 0.1 ml of 10 mM arginine solution, 0.1 ml of 0.75 mM ferrous sulfate solution, 0.4 ml of 50 mM HEPES buffer (pH 8.0) , 0.1 ml of ethylene-producing enzyme fraction, and 0.2 ml of water is shaken in a closed tube on a reciprocating shaker at 25°C for 10 minutes. The reactivity is enhanced when D.T.T. (dithiothreitol) is added in a final concentration of 1 mM.

The above experimental results suggest that the pathway of ethylene biosynthesis in P. digitatum IFO 9372 is that assimilable carbon compounds give $\alpha$-ketoglutaric acid via TCA cycle and, then, ethylene is directly produced from this $\alpha$-ketoglutaric acid in the presence of arginine and a divalent iron salt.

However, this pathway of ethylene biosynthesis holds true in P. digitatum IFO 9372 but as shown in Table 2 hereinbefore, many other fungi produce ethylene via a methionine pathway responsive to added L-methionine. Further, even organisms of the same genus Penicillium may not necessarily utilize the $\alpha$-ketoglutaric acid pathway and are often diverse according to species. Further, even among strains of P. digitatum, there are differenceS in enzyme activity for production of ethylene from $\alpha$-ketoglutaric acid and large differences in ethylene production rate. These facts are evident from the following experimental results.

Experiment 9 Ethylene production pathway in organisms of the genus Penicillium

Strains of the genus Penicillium were grown in the modified NB medium and cultural conditions mentioned in Table 1 in the routine manner and, then, maintained under starving conditions for 4 days. To the washed resting cells were added L-methionine and L-glutamic acid and the sealed reaction was conducted in the routine manner. On the other hand, cell-free extracts (corresponding to [A] in Table 5) were prepared from the cells cultured in the above modified NB medium. Each of them was reacted with substrate $\alpha$-ketoglutaric acid and the ethylene production rates were determined. The results are shown in Table 10. According to results of this experiment, none of the strains of Penicillium used, excepting P. digitatum IFO 9372, produced ethylene through $\alpha$-ketoglutaric acid ($\alpha$-KG) (L-glutamic acid in the case of resting cells).

Table 10    Biosynthesis of ethylene in Penicillium species

| Strain of Penicillium | Resting cells after culture in modified NB medium and 4-day starvation* (nl/ml broth/hr) | | | Cell-free extract of cells grown in modified NB medium** (nl/mg protein/hr) | |
|---|---|---|---|---|---|
| | No addition | L-Met added | L-GA added | No addition | α-KG and arginine added |
| P. spinulosum IFO 4686 | 1.0 | 1.0 | 0.4 | 3.5 | 3.5 |
| P. expansum IFO 5453 | 0 | 0 | 0 | 1.8 | 1.4 |
| P. viridicatum IFO 7235 | 0.1 | 0.1 | 0.1 | 0.1 | 0.1 |
| P. digitatum IFO 9372 | 2.8 | 1.3 | 8.8 | 8.0 | 588 |

* Concentration of resting cells: 18 mg/ml (final concentration in reaction mixture)

  Concentration of the additive: 1 mM (final concentration in reaction mixture)

  Reaction conditions: 25°C, reciprocating shaker method, 24 hrs.

** Cell-free extract: Final protein concentration in reaction mixture: 1 mg/ml

  Concentration of additives: α-KG: 1 mM; L-arginine: 1 mM; Fe$^{++}$: 0.075 mM

  Reaction conditions: 25°C, reciprocating shaker method, 10 min.

Experiment 10 Differences in ethylene production rate among strains of P. digitatum

While P. digitatum is a well-known ethylene-producing microorganism, its inter-strain differences were studied by growing P. digitatum IFO 7758, IFO 7006, IFO 7876, IFO 9392 and IFO 9372 in the routine manner using the modified NB medium and cultural conditions of Table 1 and comparing their ethylene production rates. The results are set forth in Table 11. Incidentally IFO 7006 is the same strain as ATCC 10030 mentioned in the literature [12] and the ethylene production rate calculated from the literature value is about 360 nl/ml broth/hr as mentioned in Table 11. However, when the present inventors carried out an experimental culture of this strain, the ethylene production rate was as low as 18 nl/ml/hr. In any event, there is a large variation in ethylene production rate among strains evident in Table 11 and the cause of these differences seems to be associated with the ability to produce α-ketoglutaric acid from carbohydrates,

14

ethylene-producing enzyme activity, and arginine-producing ability which are possessed by the respective strains.

Table 11 Comparison among strains of P. digitatum

| P. digitatum strain No. | Ability to produce α-KG from carbohydrates | Enzyme activity to produce ethylene from α-KG | Arginine-producing ability | Ethylene production rate |
|---|---|---|---|---|
| | | | | nl/ml broth/hr |
| IFO 7758 | + | + | + | 1.0 |
| IFO 7006* (ATCC10030**) | + | + | + | 18.0 (about 360 **) |
| IFO 9392 | + | ++ | + | 90.0 |
| IFO 7876 | + | +++ | + | 480.0 |
| IFO 9372 | ++ | ++++ | + | 1394 |

(Note) Grown for 4 days using the modified NB medium and cultural conditions described hereinbefore in Table 1.

* IFO 7006 is the same strain as ATCC 10030 mentioned in the literature.[12]

** The ethylene production rate based on data given in the literature.[12]

Referring to Table 11, the ability to produce α-ketoglutaric acid from carbohydrates was investigated by assaying the acid in the cells by paper chromatography and, when necessary, by combination of paper

15

chromatography and Friedemann-Haugen colorimetry. As to the enzyme activity to produce ethylene from α-ketoglutaric acid, mentioned in Table 11, the activity was determined in the aforementioned in vitro ethylene production system using α-ketoglutaric acid as the substrate. Regarding the test for arginine-producing ability in Table 11, the cell-free extract [A] in Table 5 was fractionated by high performance liquid chromatography and analyzed by fluorometry.

Based on the above experimental results, it appears that the following conditions must be satisfied in order to produce a large quantity of ethylene as in the case of P. digitatum IFO 9372.

(1) The strain must have a strong ability to produce α-ketoglutaric acid from assimilable carbon sources. As is apparent from the experimental results mentioned above, whereas ethylene biosynthesis involving methionine produces only a small amount of ethylene, ethylene biosynthesis via α-ketoglutaric acid makes for a large output of ethylene.

(2) The strain should have a high enzyme activity to produce ethylene from α-ketoglutaric acid. As is apparent from Table 11, the rate of ethylene production is remarkably dependent on the potency of this enzyme activity.

(3) The strain should have a strong arginine-producing ability. The experimental results set forth in Table 9, the results found in the in vitro system of conversion from α-ketoglutaric acid to ethylene, and the experimental results set forth in Table 11 above are not sufficient to clarify the mechanism of action of arginine. However, it is evident that arginine is an essential factor in the production of ethylene from α-ketoglutaric acid.

(4) The medium should contain a divalent iron salt. It is apparent from the experimental results set forth in Table 7 and the in vitro system of ethylene production from α-ketogluteric acid that the presence of a divalent iron salt is an essential requirement.

(5) The cultural conditions should be aerobic. The ethylene-producing strains used in the foregoing experiments were invariably aerobic microorganisms and it is, therefore, clear that oxygen must be made available during their culture but the experimental data obtained indicate that the presence of oxygen is also an essential requisite in the enzymatic reaction for the production of ethylene from α-ketoglutaric acid.

The production of ethylene in appreciably large quantities is only made possible by growing an ethylene-producing strain having the above three physiological properties (1) to (3) under the above conditions (4) and (5).

Based on the above findings, the present invention is directed to a microbiological method of producing ethylene characterized by growing aerobically a fungal strain capable of producing ethylene from α-ketoglutaric acid in the presence of L-arginine in a medium containing at least 20 ppm of divalent iron ion to produce ethylene and harvesting the ethylene.

In accordance with the present invention, there is employed a fungal strain capable of producing ethylene from α-ketoglutaric acid in the presence of L-arginine.

Preferably, this strain has a strong ability to produce α-ketoglutaric acid from carbon sources, high enzyme activity to produce ethylene from α-ketoglutaric acid, and an ability to produce arginine.

The above physiological characteristics cannot be ascribed to any genus or species and even vary among strains of the same species. Therefore, the above-mentioned strain should not be defined in taxonomic terms in the field of microbiology but be defined in physiological characteristic possessed by individual strains. Suitable strains can be identified by the procedures disclosed in the foregoing description.

Among the preferred genera of fungi is the genus Penicillium and a preferred species of the genus Penicillium is Penicillium digitatum. However, it will be obvious that the strain according to the present invention can be selected from fungi not belonging to the above-mentioned genus and species.

As the medium for cultivation of the microorganism in accordance with the present invention, a medium containing at least 20 ppm of divalent iron ion is employed.

The divalent iron ion is preferably added in the form of ferrous salt of an organic acid in the medium. When ferrous sulfate ($FeSO_4 \cdot 7H_2O$) is used as the iron salt, the preferred level of addition is within the range of 0.05 to 0.3 mM.

In the medium, the divalent iron ion may occur together with trivalent iron ion and the trivalent iron ion contained in the medium may be reduced to divalent iron ion.

As the basal medium, a conventional medium for fungi which contains carbon sources, nitrogen sources, inorganic salts and other nutrients can be employed.

Various carbohydrates such as glucose, sucrose, maltose, starch, xylose, sorbitol, etc., alcohols such as glycerol, ethanol, etc., organic acids such as acetic acid and other fatty acids, and crude materials containing them may be used as carbon sources. The main raw materials which are particularly useful for the purposes of this invention are reproducible biomasses which are either naturally occurring or available artificially as byproducts, such as materials from agricultural, forest, fishing and animal industry activities, and active sludges from the biological treatment of public sewage, raw sewage, plant effluents or various industrial wastes (enrichment cultures). Though it depends on the strains of organisms used, these main material may be dissolved, decomposed or otherwise pretreated as necessary.

As nitrogen sources, there can be advantageously used ammonia gas, aqueous ammonia and ammonium salts. When a biomass is used as the main raw material, the addition of such nitrogen sources may not be essential.

As inorganic salts, phosphates, potassium salts, magnesium salts, sodium salts, calcium salts, etc. can be routinely employed, although these may be dispensed with when a biomass is employed.

The addition of vitamins and amino acids or of materials containing them such as yeast extract, peptone, meat extract, corn steep liquor, etc. may contribute to accelerated growth of the strain used or improved yields of ethylene.

The cultivation of microorganisms is carried out under aerobic conditions, for example by aerated stirring or stationary culture, with the pH and temperature being controlled at pH 2 to 9 and 20 to 45°C, respectively. Thus, for each strain, the optimum pH and temperature are selected. As the cultivation is conducted for 1 to 10 days, a biogas containing a significant amount of ethylene is produced.

The ethylene content of the product biogas is assayed as follows.

A $x = 1$ to 5 ml portion of the broth in the course of cultivation or at the end of cultivation is taken into a test tube with a total volume of $V = 10 - 50$ ml and after closure with a sterile rubber stopper, the broth is incubated on a reciprocating shaker at 20 to 45°C for $t = 1 - 7$ hours. Since the respiration rate varies with different strains, it is preferable to vary the parameters V, x and t so as to prevent oxygen deficiency during shaking.

After the reciprocal shaking, $y = 0.1$ to 2 ml of the gas is taken from the top plenum of the test tube using a gas syringe and subjected to conventional FID gas chromatography (column temperature 50°C, injection temperature 100°C) using nitrogen gas as the carrier gas.

From the area under the FID trace the ethylene content $E^{nl}$ of the gas sample is determined against the calibration curve constructed with standard gas. The production rate of ethylene P nl/ml•hr can be calculated by means of the following equation.

$$P = E \cdot \left(\frac{V - x}{y}\right) \cdot \frac{1}{x} \cdot \frac{1}{t}$$

To separate the ethylene from the product biogas, the biogas is either adsorbed on a suitable adsorbent such as zeolite or activated carbon or contacted with a sodium hydroxide solution to remove the byproduct carbon dioxide gas and, then, absorbed on the above-mentioned adsorbent, followed by desorption. The zeolite may for example be Molecular Sieves 4A [Union Showa K. K.] or Zeoram A-4, A-5 or F-9 [Toyo Soda Industries, Ltd.]. The activated carbon may for example be Molecular Sieving Carbon [Takeda Chemical Industries, Ltd.].

The following examples are intended to illustrate the present invention in further detail.

Example 1

The fungal strains preserved after allotment and those newly alloted from a stock culture collection were cultivated in the modified NB medium of Table 1 in the routine manner. Then, using the strains showing high ethylene production rates, their physiological characteristics were investigated in accordance with Table 11 and the strains meeting the requirements set forth hereinbefore were selected. These strains and their characteristics are shown in Table 12. Thus, other than Penicillium digitatum IFO 9372, there were obtained 3 ethylene-producing strains which were presumed to produce ethylene via α-ketoglutaric acid.

For the purpose of verifying the route of ethylene production, the investigation according to Table 10 was conducted. The results are shown in Table 13. It was confirmed that all of the strains produce ethylene via α-ketoglutaric acid.

17

Table 12 Physiological characteristics of fungi capable
of producing large amounts of ethylene

| Selected fungal strain | Ability to produce α-KG from carbo-hydrates | Enzyme activity to produce ethylene from α-KG | Ability to produce ariginine | Ethylene production rate |
|---|---|---|---|---|
| | | | | (nl/ml broth/hr.) |
| Chaetomium globosum IFO 6347 | + | ++ | + | 24.6 |
| Phycomyces nitens IFO 9422 | + | ++ | + | 42.9 |
| Paecilomyces carneus IFO 8292 | + | ++ | + | 37.5 |
| Penicillium digitatum IFO 9372 (parent) | ++ | ++++ | + | 1394 |
| Penicillium digitatum IFO 9372-R15* | ++ | +++++ | + | 2114 |

Each strain was grown in the modified NB medium of Table 1 and
the parameters were determined in accordance with Table 11.

*: A small colony strain obtained by monospore isolation
from P. digitatum.

EP 0 205 303 B1

Table 13 Production of ethylene by fungi capable of producing large amounts of ethylene

| | Resting cells after cultivation in modified NB medium and subsequent 4-day starvation* | | | Cell-free extract of cells grown in modified NB medium** | |
|---|---|---|---|---|---|
| | No addition | L-Met added | L-GA added | No addition | α-KG and arginine added |
| | (nl/ml broth/hr) | | | (nl/mg protein/hr) | |
| Chaetomium globosum IFO 6347 | 0.2 | 0.2 | 2.6 | 2.3 | 13.9 |
| Phycomyces nitens IFO 9422 | 0.3 | 0.3 | 4.2 | 1.9 | 21.5 |
| Paecilomyces carneus IFO 8292 | 0.1 | 0.1 | 2.0 | 1.2 | 7.4 |
| Penicillium digitatum IFO 9372 (parent) | 2.8 | 1.3 | 8.8 | 8.0 | 588 |
| Penicillium digitatum IFO 9372-R15 | 4.3 | 1.3 | 15.5 | 8.5 | 1302 |

*, **: The determinations were made under the same conditions as Table 10.

Example 2

The influence of addition levels of a divalent iron salt to the medium for Penicillium digitatum IFO 9372 and the influence of oxygen partial pressure on ethylene production were investigated. The results are given in Tables 14 and 15, respectively.

With an increasing concentration of the divalent iron salt in the medium, the ethylene production rate increased, and as the oxygen partial pressure at ethylene producing reaction was zero or anoxic, production of ethylene ceased to occur.

Table 14

| Influence of addition levels of a divalent iron salt to the medium for P. digitatum IFO 9372 | |
| --- | --- |
| Level of addition of $FeSO_4 \cdot 7H_2O$ | Ethylene production rate (nl/ml broth/ hr) |
| 1.0 g/ℓ | 135 |
| 0.33 | 123 |
| 0.10 | 110 |
| 0.033 | 100 |
| 0.01 | 90 |
| 0.0033 | 60 |
| 0 | 2 |

Medium used: Calculated amounts of $FeSO_4 \cdot 7H_2O$ were added to the synthetic medim for fungi (See Table 1) minus $FeSO_4 \cdot 7H_2O$.

Cultural conditions: 25°C, rotary shaker method, 5 days

Table 15

| Influence of oxygen partial pressure in the sealed reaction of P. digitatum IFO 9372 | |
| --- | --- |
| Oxygen partial pressure (The remainder nitrogen gas) | Ethylene production rate (nl/ml broth/hr) |
| 60% | 1,108 |
| 40% | 1,140 |
| 20% | 1,230 |
| 10% | 1,185 |
| 5% | 1,207 |
| 0% | 0 |

Medium used: Modified NE medium

Cultural conditions: 25°C, rotary shaker method, 4 days

Sealed reaction: The cells on the 4th day of culture were exposed to the respective oxygen partial pressures. The sealed reaction was conducted for 2 hrs.

Example 3

A miniature fermentation jar of 2.6 ℓ capacity was charged with 1 ℓ of the modified NB medium of Table 1 and sterilized by autoclaving at 120°C for 20 minutes. After cooling, 50 ml of a culture of Penicillium digitatum IFO 9372 shake-cultured in the same medium as above was transplanted into the sterilized medium and incubated at 25°C and 400 rpm whilst purging with 0.1VVM of germ-free air for 6 days.

Throughout this incubation period, the discharge gas was passed through a 10% sodium hydroxide bath, water stripping bath and moisture separation bath in that order to remove impurity gases. The gas was further passed through a bed of Zeolam A-4 [Toyo Soda] to further adsorb and remove impurity gases and the effluent gas was guided into a column of Zeolam A-4 [Toyo Soda] and the adsorbed ethylene was desorbed by vacuum suction for recovery. The yield of ethylene was about 100 mg.

The present invention is characterized in that the readily-available, reproducible biomass, particularly the waste resources from agricultural, forest, fishery, animal husbandry and other industries and/or sludges available from the biological treatment of public sewage, raw sewage, plant effluents, industrial wastes, etc. can be advantageously utilized as main raw materials and that the ethylene fermentation according to the present invention as such is a microbiological treatment of wastes and effluents as raw material biomass. Furthermore, the method according to the present invention is advantageous over the conventional methods for ethylene production in that the main raw material may be a reproducible biomass which will never be depleted, the production of ethylene is accomplished under comparatively low or mild temperature and pressure conditions because of its being a microbiological process, and the byproduct gas contained in the

20

EP 0 205 303 B1

product biogas is mostly carbon dioxide gas so that the purification of ethylene is easier and the purity of product ethylene is high. Recently, it has been attempted to carry out an alcohol fermentation using a reproducible biomass and a microorganism and, then, convert the alcohol chemically into ethylene. However, the method according to this invention is a one-step process, thus enabling one to produce ethylene in a single fermentation step.

**Claims**

1. A microbiological method of producing ethylene which comprises growing aerobically a fungal strain having an ability to elaborate ethylene from $\alpha$-ketoglutaric acid in the presence of L-arginine in a medium containing at least 20 ppm of divalent iron to produce ethylene and recovering ethylene thus produced.

2. A method as claimed in claim 1 wherein the fungal strain has a strong ability to produce $\alpha$-ketoglutaric acid from assimilable carbon sources, a high enzymatic activity to produce ethylene from $\alpha$-ketoglutaric acid, and ability to produce arginine.

3. A method as claimed in claim 1 or claim 2 wherein the strain belongs to the genus Chaetomium, Phycomyces, Paecilomyces or Penicillium.

4. A method as claimed in claim 3 wherein the strain is that of Chaetomiun globosum, Phycomyces nitens, Paecilomyces carneus or Penicillium digitatum.

5. A method as claimed in claim 4 wherein the strain is Penicillium digitatum ATCC 10030, IFO 7876 or IFO 9372.

**Revendications**

1. Procédé microbiologique de production d'éthylène, qui consiste à cultiver de manière aérobie une souche de champignons ayant la capacité d'élaborer de l'éthylène à partir de l'acide $\alpha$-cétoglutarique en présence de L-arginine dans un milieu contenant au moins 20 ppm de fer divalent pour produire de l'éthylène, et à recueillir l'éthylène ainsi produit.

2. Procédé selon la revendication 1, dans lequel la souche de champignons a une forte capacité de production d'acide $\alpha$-cétoglutarique à partir de sources de carbone assimilable, une activité enzymatique élevée de production d'éthylène à partir d'acide $\alpha$-cétoglutarique et une capacité de production d'arginine.

3. Procédé selon la revendication 1 ou 2, dans lequel la souche appartient aux genres *Chaetomium*, *Phycomyces, Faecilomyces* ou *Penicillium*.

4. Procédé selon la revendication 3, dans lequel la souche est *Chaetomium globosum, Phycomyces nitens*, *Paecilomyces carneus* ou *Penicillium digitatum*.

5. Procédé selon la revendication 4, dans lequel la souche est *Penicillium digitatum* ATCC 10030, IFO 7876 ou IFO 9372.

**Patentansprüche**

1. Ein mikrobiologisches Verfahren zur Herstellung von Ethylen, bei dem man eine Pilzart aerobisch wachsen läßt, welche die Fähigkeit besitzt, Ethylen aus $\alpha$-Ketoglutarsäure in Gegenwart von L-Arginin in einem Medium, welches wenigstens 20 ppm zweiwertigen Eisens enthält, zu bilden, um Ethylen herzustellen und auf diese Weise hergestelltes Ethylen zurückzugewinnen.

2. Verfahren wie in Anspruch 1 beansprucht, bei dem die Pilzart eine ausgeprägte Fähigkeit besitzt, $\alpha$-Ketoglutarsäure aus assimilierbaren Kohlenstoffquellen herzustellen, eine hohe enzymatische Aktivität besitzt, um Ethylen aus $\alpha$-Ketoglutarsäure zu bilden und die Fähigkeit besitzt, Arginin herzustellen.

21

3. Verfahren wie in Anspruch 1 oder Anspruch 2 beansprucht, bei dem die Art der Gattung Chaetomium, Phycomyces, Paecilomyces oder Penicillium angehört.

4. Verfahren wie in Anspruch 3 beansprucht, bei dem die Art Chaetomium globosum, Phycomyces nitens, Paecilomyces carneus oder Penicillium digitatum ist.

5. Verfahren wie in Anspruch 4 beansprucht, bei dem die Art Penicillium digitatum ATCC 10030, IFO 7876 oder IFO 9372 ist.